# FASCICULE DE BREVET EUROPEEN

(11) **EP 3 498 685 B1**
(45) Date de publication et mention de la délivrance du brevet: **28.10.2020**
(21) Numéro de dépôt: 18209433.4
(22) Date de dépôt: 30.11.2018
(51) Int. Cl.: C07C 7/12, C07C 7/05, C07C 11/08, C07C 11/09, C10G 53/08

(54) **PROCEDE D'ELIMINATION SIMULTANEE D'ISOBUTANAL ET D'ETHANOL DE CHARGES OLEFINIQUES PAR ADSORPTION SUR UN MATERIAU A BASE D'OXYDE REFRACTAIRE POREUX**
VERFAHREN ZUR GLEICHZEITIGEN ABSONDERUNG VON ISOBUTANAL UND ETHANOL AUS OLEFINISCHEN LADUNGEN DURCH ADSORPTION EINES MATERIALS AUF DER BASIS VON PORÖSEM HITZEBESTÄNDIGEM OXID
METHOD FOR SIMULTANEOUS ELIMINATION OF ISOBUTANOL AND ETHANOL FROM OLEFIN FEEDSTOCKS BY ADSORPTION ON A MATERIAL MADE OF POROUS REFRACTORY OXIDE

(30) Priorité: 13.12.2017 FR 1762091
(43) Date de publication de la demande: 19.06.2019
(73) Titulaire: IFP Energies nouvelles, 92852 Rueil-Malmaison Cedex (FR); Total Research & Technology Feluy, 7181 Seneffe (BE)
(72) Inventeur: BARTHELET, Karin, 69007 Lyon (FR); BRACCO, Emmanuelle, 69420 Condrieu (FR); COUPARD, Vincent, 69100 Villeurbanne (FR); NESTERENKO, Nikolai, 1400 Nivelles (BE)
(74) Mandataire: IFP Energies nouvelles

(56) Documents cités:
- EP-B1- 2 547 639

## Description

### Domaine de l'invention

La présente invention concerne un procédé de purification d'une charge oléfinique comprenant une étape d'élimination simultanée d'aldéhydes et d'alcools contenus dans la charge oléfinique, cette étape étant opérée à basse température (inférieure ou égale à 200°C) par adsorption sur un adsorbant comprenant un support poreux essentiellement à base d'oxyde réfractaire poreux, comme l'alumine. Plus particulièrement, l'invention concerne un procédé de purification d'une charge oléfinique comprenant des oléfines à 4 atomes de carbone et des impuretés comprenant l'isobutanal, l'éthanol et l'acétone, ledit procédé comprenant une étape d'élimination simultanée d'isobutanal et d'éthanol par passage de ladite charge oléfinique, préalablement traitée, sur un lit fixe comprenant au moins un matériau à base d'oxyde réfractaire poreux, éventuellement imprégné d'un ou plusieurs cations alcalins ou alcalino-terreux. Le pré-traitement de la charge oléfinique comprend au moins l'élimination de l'acétone et éventuellement de l'eau, présentes dans la charge oléfinique.

L'invention s'applique avantageusement au traitement des effluents de conversion de composés oxygénés et notamment au traitement de l'effluent issu de la déshydratation d'un isomère du butanol , notamment de l'isobutanol, ou d'un mélange d'isomères du butanol comprenant de l'isobutanol.

### Etat de la Technique

Les butènes produits par déshydratation d'alcool, en particulier par déshydratation d'un isomère du butanol, notamment de l'isobutanol, ou d'un mélange d'isomères du butanol comprenant de l'isobutanol, contiennent généralement quelques centaines de ppm massiques d'isobutanal, d'éthanol et d'acétone. Or pour respecter les spécifications imposées aux butènes, il s'avère nécessaire de les éliminer. En effet, les butènes produits peuvent être destinés à être envoyés dans une unité de métathèse qui utilise un catalyseur qui ne tolère pas plus de 10 ppm poids en oxygène. On entend par ppm poids en oxygène ou teneur en oxygène, la teneur massique en atome d'oxygène contenu dans le mélange considéré. Cette teneur en oxygène peut être calculée à partir de la composition massique ou molaire en composés hydrocarbonés oxygénés du mélange considéré.

Le brevet EP 2 547 639 B1, qui décrit un procédé de métathèse d'alcènes, explique ainsi que de nombreux polluants, présents dans la charge de métathèse, sont responsables de la chute d'activité du catalyseur.

Pour atteindre des niveaux de concentration en impuretés très faibles, une des techniques souvent utilisée est l'adsorption sur un solide mis en œuvre dans un lit fixe. Il s'agit néanmoins de trouver le ou les bons adsorbants pour permettre l'élimination de toutes les impuretés présentes.

Le brevet EP 2 547 639 B1 cite les alumines et zéolithes comme adsorbants potentiels. Cependant, aucun lien entre la nature des composés à éliminer et le choix de l'adsorbant pour obtenir un traitement efficace des alcènes n'est donné.

L'article C. C. Brunchi et. al., Ind. Eng. Chem. Res., 2012, 51, 16697-16708, décrit la co-adsorption en batch d'un mélange de composés organiques volatils (VOC), le butanal, le 2-éthyl-2-hexanal, le 2,6-diméthylcyclohexanone, le 2,4,6-triméthylphénol et le 2,4,6-triméthylanisole, dans du toluène. Il apparait que la zéolithe NaY permet d'adsorber des quantités significatives de ces différentes molécules. C. C. Brunchi *et. al.* montrent également que la capacité de captation de la zéolithe diminue très fortement avec la baisse de la concentration en composés dans le toluène. La mise en œuvre de ce type de solide rendra l'efficacité du procédé dépendante de la concentration de la charge, ce qui rend le dimensionnement de la zone de purification délicate. C. C. Brunchi *et. al.* ont également évaluer des alumines pour éliminer les VOC par adsorption : ils montrent que seul le butanal est adsorbable en quantité importante. Les capacités de captation de ces alumines des autres composés organiques sont faibles.

La Demanderesse a mis en évidence que la mise en œuvre en lit fixe d'au moins un adsorbant comprenant au moins un matériau à base d'oxyde réfractaire poreux éventuellement imprégné d'un ou plusieurs cations alcalins ou alcalino-terreux, permet d'éliminer simultanément l'isobutanal et l'éthanol de charges oléfiniques comprenant des oléfines à 4 atomes de carbone, desquelles l'acétone et éventuellement l'eau ont été préalablement éliminées.

La présente invention concerne ainsi un procédé de purification d'une charge oléfinique comprenant des oléfines à 4 atomes de carbone, en particulier issue de la déshydratation d'un isomère du butanol, notamment de l'isobutanol, ou d'un mélange d'isomères du butanol comprenant de l'isobutanol, permettant l'élimination des impuretés isobutanal, acétone et éthanol, contenues à hauteur de quelques centaines de ppm massique dans ladite charge.

### Résumé de l'invention.

En particulier, la présente invention concerne un procédé de purification d'une charge oléfinique comprenant des oléfines à 4 atomes de carbone et des impuretés incluant l'isobutanal, l'éthanol et l'acétone, ledit procédé comprenant :
a) une étape de pré-traitement comprenant au moins une étape d'élimination de l'acétone ; et
b) une étape d'élimination simultanée de l'isobutanal et de l'éthanol, par passage de la charge pré-traitée issue de l'étape a) sur au moins un lit fixe d'au moins un adsorbant comprenant au moins un matériau à base d'oxyde réfractaire poreux,
ladite étape b) opérant à une température comprise entre 0 et 200°C, à une pression de 0,1 à 10 MPa et avec une vitesse volumique horaire (VVH) de la charge pré-traitée issue de l'étape a) sur le lit fixe comprise entre 0,1 et 10 h⁻¹.

L'invention s'applique avantageusement au traitement des effluents de conversion de composés oxygénés et notamment au traitement de l'effluent issu de la déshydratation d'un isomère du butanol, notamment de l'isobutanol, ou d'un mélange d'isomères du butanol comprenant de l'isobutanol. En particulier, l'invention s'applique au traitement de l'effluent issu de la déshydratation de l'isobutanol seul ou en mélange avec d'autres isomères du butanol.

Avantageusement, l'utilisation d'un lit fixe d'au moins un adsorbant spécifique, ledit adsorbant comprenant au moins un matériau à base d'oxyde réfractaire poreux, dans les conditions particulières selon l'invention, permet d'éliminer simultanément les impuretés isobutanal et éthanol contenues dans le mélange de butènes issus de la déshydratation de l'isobutanol ou d'un mélange d'isomères du butanol comprenant de l'isobutanol.

Le procédé de purification selon l'invention permet ainsi d'obtenir, grâce notamment à l'utilisation d'un lit fixe d'adsorbant spécifique et à des conditions optimisées, une teneur en oxygène, indicatrice de la teneur en impuretés hydrocarbonées oxygénées dans le mélange d'oléfines à l'issue de la purification, inférieure à 50 ppm massique, préférentiellement inférieure ou égale à 30 ppm massique, de préférence inférieure ou égale à 15 ppm massique, de manière préférée inférieure ou égale à 10 ppm massique et de manière très préférée inférieure ou égale à 1 ppm massique. On entend par teneur en oxygène, la teneur massique en atome d'oxygène contenu dans le mélange considéré. Cette teneur en oxygène peut être calculée à partir de la composition massique ou molaire en composés hydrocarbonés oxygénés du mélange considéré. Avantageusement, le procédé selon l'invention permet d'obtenir des teneurs massiques en impuretés hydrocarbonées oxygénées, telles que l'isobutanal, l'éthanol et l'acétone, dans le mélange d'oléfines à l'issue de la purification, inférieures à 50 ppm massique, de préférence inférieures ou égales à 30 ppm massique, préférentiellement inférieures ou égales à 15 ppm massique, de manière préférée inférieures ou égales à 10 ppm massique et de manière très préférée inférieures ou égales à 1 ppm massique.

A la différence des adsorbants de type zéolithique dont la sélectivité est très favorable à l'éthanol, l'adsorbant comprenant au moins un matériau à base d'oxyde réfractaire poreux peut réaliser le traitement de charges oléfiniques contenant de l'isobutanal et de l'éthanol, sans que ce dernier n'empêche l'adsorption de l'isobutanal.

Dans le procédé selon l'invention, l'adsorbant comprenant au moins un matériau à base d'oxyde réfractaire poreux peut subir plusieurs cycles de régénération et conserver des capacités de captation en isobutanal et en éthanol satisfaisantes, ce qui peut présenter un réel avantage économique.

### Description de l'invention

Dans la présente invention, on entend par « teneur en impuretés hydrocarbonées oxygénées » ou « teneur massique en impuretés hydrocarbonées oxygénées » d'une charge ou d'un effluent (charge initiale, charge oléfinique ou effluent en sortie de procédé) la masse d'impuretés hydrocarbonées oxygénées, telles que l'isobutanal, l'éthanol et l'acétone, par unité de masse de la charge ou de l'effluent considéré. On citera par exemple comme technique usuelle de détermination de la teneur en chacune des impuretés hydrocarbonées oxygénées la chromatographie en phase gazeuse correspondant à la détermination selon la méthode UOP 960.

Il faut noter que l'oxygène de l'eau présente sous forme dissoute dans la charge ou l'effluent n'est pas comptabilisé dans la teneur en impuretés hydrocarbonées oxygénées. La teneur en eau présente sous forme dissoute dans la charge ou l'effluent peut être déterminée par une technique spécifique, par exemple selon la méthode Karl Fischer (cf. Analyse des solvants résiduels dans les produits pharmaceutiques, techniques de l'ingénieur P3260v1 M.Bauer 10/09/2001).

Dans la présente invention, on entend par « vitesse volumique horaire (VVH) de la charge sur le lit fixe » le ratio entre le débit volumique horaire de la charge à traiter et le volume du réacteur.

Selon la présente invention, l'expression « compris entre ... et ... » signifie que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

La présente invention consiste en un procédé de purification d'une charge oléfinique comprenant des oléfines à 4 atomes de carbone et des impuretés incluant l'isobutanal, l'éthanol et l'acétone, ledit procédé comprenant :
a) une étape de pré-traitement comprenant au moins une étape d'élimination de l'acétone ; et
b) une étape d'élimination simultanée de l'isobutanal et de l'éthanol, par passage de la charge pré-traitée issue de l'étape a) sur au moins un lit fixe d'au moins un adsorbant comprenant au moins un matériau à base d'oxyde réfractaire poreux ;
ladite étape b) opérant à une température comprise entre 0 et 200°C, à une pression de 0,1 à 10 MPa et avec une vitesse volumique horaire (VVH) de la charge pré-traitée issue de l'étape a) sur le lit fixe comprise entre 0,1 et 10 h⁻¹.

### La charge oléfinique

La charge oléfinique traitée par le procédé de purification selon l'invention est un mélange d'oléfines. Ce mélange peut contenir des oléfines linéaires, ramifiées ou un mélange d'oléfines linéaires et ramifiées.

La charge oléfinique est caractérisée par sa teneur très élevée en oléfines. Elle contient de préférence au moins 95% poids, préférentiellement au moins 97% poids de son poids sec en oléfines. Le titre élevé en oléfines donne à cette coupe une réactivité particulièrement élevée, autant dans les étapes de valorisation avales, que dans les étapes de transformation ou purification qu'elle subit.

Les oléfines présentes dans la charge sont des composés principalement à 4 atomes de carbones, plus particulièrement des butènes, notamment un mélange de n-butènes et d'isobutène.

La charge traitée selon l'invention peut également contenir des composés non oléfiniques parmi lesquels peuvent se trouver, en plus de l'eau, des composés organiques hydrocarbonés (impuretés). Les composés organiques hydrocarbonés peuvent être des paraffines et des diènes, des composés organiques oxygénés parmi lesquels on peut citer les aldéhydes, les cétones, les alcools, les acétals, les éthers esters, les furanes, les acides carboxyliques.

Plus particulièrement, la charge traitée selon l'invention comprend des oléfines à 4 atomes de carbone et des impuretés hydrocarbonées oxygénées comprenant l'isobutanal, l'éthanol et l'acétone.

Selon une variante de l'invention, la charge oléfinique est issue de la conversion de composés oxygénés. De préférence, la charge oléfinique traitée selon l'invention est issue de la déshydratation de l'isobutanol ou d'un mélange d'isomères du butanol comprenant de l'isobutanol. Préférentiellement, la charge oléfinique traitée selon l'invention est issue de la déshydratation d'un isomère du butanol, en particulier l'isobutanol, ou d'un mélange d'isomères du butanol comprenant l'isobutanol. Typiquement, les butènes produits par déshydratation de l'isobutanol ou d'un mélange d'isomères du butanol comprenant de l'isobutanol, contiennent quelques centaines de ppm d'isobutanal, d'éthanol et d'acétone.

### Etape a)

Conformément à l'invention, la charge oléfinique subit un pré-traitement comprenant au moins une étape d'élimination de l'acétone. Le pré-traitement peut en outre comprendre une étape d'élimination de l'eau que pourrait contenir la charge oléfinique.

Il a été trouvé par la Demanderesse que parmi les impuretés présentes dans la charge oléfinique, certaines sont incompatibles avec l'utilisation d'un adsorbant à base d'oxydes réfractaires poreux. En particulier, la présence d'acétone dans la charge réduit notablement les performances de l'adsorbant comprenant au moins un matériau à base d'oxyde réfractaire poreux, comme l'alumine. En effet, l'acétone réagit avec l'isobutanal au contact du matériau à base d'oxyde réfractaire qui comporte notamment des sites de nature basique (comme les alumines, MgO,...), pour donner des composés plus lourds, moins bien adsorbés et qui réduisent ainsi la capacité d'adsorption et également l'accès aux sites d'adsorption. Il convient donc d'éliminer ces impuretés, en particulier l'acétone, préalablement au passage de la charge à traiter sur l'adsorbant comprenant au moins un matériau à base d'oxyde réfractaire poreux, éventuellement dopé.

Le pré-traitement permet ainsi de limiter, voire d'empêcher la réaction de l'isobutanal avec l'acétone (aldolisation). Il permet aussi d'éviter la perte de capacité d'adsorption de l'adsorbant utilisé à l'étape b) qui comprend le matériau à base d'oxyde réfractaire poreux, vis-à-vis de l'isobutanal et de l'éthanol. Le pré-traitement de la charge oléfinique, selon l'étape a), permet donc d'optimiser la capacité de captation en isobutanal et éthanol de l'adsorbant comprenant le matériau à base d'oxyde réfractaire, à l'étape b).

L'acétone et éventuellement l'eau contenues dans la charge oléfinique sont éliminées par toute méthode connue de l'homme du métier. En particulier, l'acétone peut être enlevée de la charge à traiter par des méthodes de stripage, distillation, étêtage, pasteurisation ou rectification. L'eau peut être éliminée, par exemple, par adsorption sur tamis moléculaire type 3A.

Lorsqu'elle est intégrée au procédé de traitement selon l'invention, l'étape d'élimination de l'eau peut être réalisée simultanément à l'étape d'élimination de l'acétone. Elle peut aussi être réalisée précédemment ou successivement à l'étape d'élimination de l'acétone.

Avantageusement, à l'issue du pré-traitement (étape a), la charge oléfinique contient moins de 10 ppm massique, de préférence moins de 1 ppm massique d'eau, et moins de 10 ppm massique, de préférence moins de 1 ppm massique d'acétone. Et plus particulièrement, elle est dépourvue d'eau et d'acétone.

### L'adsorbant à base d'oxyde réfractaire poreux

Conformément à l'invention, le procédé de purification comprend le passage de la charge oléfinique dans un réacteur comprenant au moins un lit fixe d'au moins un adsorbant spécifique. L'adsorbant selon l'invention comprend au moins un matériau à base d'oxyde réfractaire poreux.

Le matériau à base d'oxyde réfractaire poreux est de préférence choisi dans le groupe consistant en : l'alumine, la silice, l'oxyde de titane, l'oxyde de zirconium, l'oxyde de magnésium et leurs mélanges. De préférence, le matériau à base d'oxyde réfractaire poreux est à base d'alumine.

Les oxydes réfractaires poreux préférés ont une surface spécifique, mesurée par la méthode B.E.T selon la norme ASTM D3663, exprimée en S_{BET}, avantageusement comprise entre 30 m²/g et 400 m²/g.

Les oxydes réfractaires poreux préférés présentent un volume poreux total, déterminé par intrusion au mercure selon la norme ASTM D6761 avec un angle de mouillage de 140°, par exemple au moyen d'un appareil modèle Autopore III™ de la marque Microméritics™, supérieur ou égal à 0,05 ml/g, de préférence compris entre 0,1 et 1,5 ml/g.

Avantageusement, les oxydes réfractaires présentent une macroporosité. Plus particulièrement, les oxydes réfractaires poreux préférés présentent un volume macroporeux, c'est-à-dire le volume cumulé des pores de diamètre supérieur à 50 nm, supérieur ou égal à 0,01 ml/g, de préférence compris entre 0,01 et 0,4 ml/g et de manière très préférée compris entre 0,05 et 0,2 ml/g. Ce volume est déterminé par intrusion de mercure selon la norme ASTM D4284 avec un angle de mouillage de 140°, par exemple au moyen d'un appareil modèle Autopore III™ de la marque Microméritics™.

Le matériau à base d'oxyde réfractaire poreux est éventuellement imprégné d'un ou plusieurs cations alcalins ou alcalino-terreux.

Les cations alcalins sont des cations d'élément choisi parmi le lithium, le sodium, le potassium, le rubidium et le césium, de préférence parmi le sodium et le potassium. Les cations alcalino-terreux sont des cations d'élément choisi parmi le béryllium, le magnésium, le calcium, le strontium, le baryum et le radium, de préférence parmi le magnésium, le calcium et le strontium et de manière encore plus préférée parmi le magnésium et le calcium.

De préférence, le matériau à base d'oxyde réfractaire poreux est imprégné d'un ou plusieurs cations alcalins ou alcalino-terreux à hauteur de 0,2% à 40% poids, de préférence de 0,2 à 30% poids, de manière préférée de 0,2 à 20% poids, de manière très préférée de 0,2 à 10% poids, par rapport au poids total de l'adsorbant après imprégnation.

La teneur en cations peut être mesurée par spectroscopie d'absorption atomique, en particulier selon la méthode décrite dans l'ouvrage de J. Lynch « Analyse Physico-chimique des catalyseurs industriels - Manuel pratique de caractérisation », Editions Technip, 2001.

L'imprégnation du matériau à base d'oxyde réfractaire poreux peut être réalisée par toutes les méthodes connues, en particulier par immersion ou à sec. De manière préférée, le ou les cations alcalins ou alcalino-terreux sont imprégnés à sec sur le matériau à base d'oxyde réfractaire poreux. L'imprégnation à sec consiste à mettre en contact une quantité d'oxyde réfractaire poreux avec un volume de solution d'imprégnation qui correspond exactement à son volume poreux disponible. Le ou les cations alcalins ou alcalino-terreux peuvent être imprégnés en une ou plusieurs fois.

Les précurseurs des cations alcalins ou alcalino-terreux sont choisis de manière préférée parmi des hydroxydes, des nitrates, des carbonates.

Après chaque imprégnation, le matériau à base d'oxyde réfractaire poreux imprégné obtenu est séché à une température comprise entre 90 et 120°C pendant 3 à 12h puis éventuellement calciné sous air à une température comprise entre 300 et 550°C pendant 0,5 à 6 h. Le séchage et la calcination peuvent se faire par exemple sous azote ou sous air sec ou humide.

Avantageusement, le matériau à base d'oxyde réfractaire poreux utilisé dans le procédé de purification selon l'invention est mis en forme. Toute méthode connue peut être utilisée pour la mise en forme du matériau à base d'oxyde réfractaire poreux.

Avantageusement, l'adsorbant est utilisé dans le procédé de purification selon l'invention sous la forme d'un empilement de particules élémentaires dissociées ou sous la forme d'un ou plusieurs monolithes multicanaux installés en série ou en parallèle.

Dans le cas où l'adsorbant est utilisé sous la forme d'un empilement de particules élémentaires dissociées, il se présente sous la forme de billes, de cylindres multilobés, de préférence avec un nombre de lobes compris entre 2 et 5 ou sous forme d'anneaux, de cylindres creux, d'anneaux creux, d'anneaux de Raschig, de cylindres creux dentelés, de cylindres creux crénelés, de roues de charrettes, de selles de Berl ou de cylindres à multiples trous, seuls ou en mélange.

Dans le cas où l'adsorbant est utilisé sous la forme d'un ou plusieurs monolithes multicanaux installés en série ou en parallèle, ledit adsorbant se présente de préférence sous la forme d'un monolithe multicanaux de type nid d'abeilles, les canaux pouvant être de section carrée, hexagonale, circulaire ou ovale. La surface des canaux du monolithe peut être lisse, cannelée ou rugueuse afin de favoriser un contact intime du fluide à traiter avec la surface des canaux du monolithe. La densité de canaux peut préférentiellement varier de 5 cpsi à 400 cpsi (cpsi = canal per square inch) (respectivement de environ 0,8 à environ 62,0 canaux par cm²). Alternativement, le monolithe peut être constitué de mousse céramique. La densité de pores de la mousse céramique est comprise entre 10 ppi et 60 ppi, et de manière préférée entre 10 et 30 ppi (ppi = pores per inch) (respectivement comprise entre environ 3,9 et environ 23,6 pores par cm et de manière préférée entre environ 3,9 et environ 11,8 pores par cm).

La section du monolithe doit être égale à la section interne du réacteur contenant le lit d'adsorbants afin de forcer l'intégralité du flux à traiter à circuler à l'intérieur des canaux du monolithe.

Avantageusement, le lit fixe peut comprendre des adsorbants présentant un ou plusieurs types de mise en forme. L'adsorbant comprenant le matériau à base d'oxyde réfractaire poreux peut être utilisé seul ou en mélange avec un ou des autre(s) adsorbant(s).

Il est particulièrement avantageux de superposer des adsorbants différents, en particulier de nature et/ou de mise en forme différentes, dans au moins deux lits fixes différents de hauteur variable, les adsorbants ayant le plus faible taux de vide, le taux de vide étant défini comme le rapport de la différence entre le volume du réacteur et le volume de solide sur le volume de réacteur (c'est-à-dire présentant le plus faible volume rempli par l'adsorbant considéré, rapporté au volume du réacteur) étant de préférence utilisés dans le ou les premiers lits fixes, en entrée du réacteur afin de réaliser une sorte de filtration de la charge lors de son passage dans le ou les lits. Des adsorbants différents peuvent également être mélangés au sein d'un même lit.

Selon un mode de réalisation de l'invention, l'adsorbant peut être activé, notamment in situ, juste avant sa première utilisation dans le procédé selon l'invention, selon l'une quelconque des méthodes connues de l'Homme du métier. Avantageusement, l'activation est réalisée par passage d'un gaz chauffé entre 100 et 500°C. Le gaz utilisé peut être un gaz de combustion, de l'air, de l'azote. De préférence le gaz d'activation est l'azote.

### Etape b)

Conformément à l'invention, le procédé de purification de la charge oléfinique, en particulier l'étape d'élimination simultanée de l'isobutanal et de l'éthanol, opère à une température comprise entre 0°C et 200°C, de préférence comprise entre 10 et 100°C et de préférence entre 20 et 60°C, par exemple à 30°C, à une pression comprise entre 0,1 et 10 MPa, de préférence comprise entre 0,3 et 5 MPa, et avec une vitesse volumique horaire VVH de ladite charge sur ledit lit fixe comprise entre 0,1 et 10 h⁻¹ et de préférence comprise entre 0,2 et 5 h⁻¹.

Dans ces conditions et grâce à l'adsorbant spécifique utilisé dans le procédé de purification selon l'invention, l'adsorption des aldéhydes et des alcools, en particulier l'isobutanal et de l'éthanol, est observée à la surface de l'adsorbant à des taux satisfaisants. Le procédé de purification selon l'invention, grâce notamment à l'adsorbant spécifique utilisé dans les conditions particulières telles que définies, permet avantageusement de capter l'isobutanal et l'éthanol dans le flux à traiter et d'obtenir un flux en sortie ayant des teneurs en isobutanal et en éthanol réduites par rapport aux teneurs du flux initial, voire d'éliminer totalement l'isobutanal et l'éthanol. En particulier, les teneurs, massiques ou molaires, en isobutanal et en éthanol dans l'effluent après traitement peuvent être diminuées d'au moins 90%, préférentiellement au moins 95%, et plus préférentiellement au moins 99% par rapport aux teneurs correspondantes dans la charge oléfinique avant traitement.

Selon un mode de réalisation préféré de la présente invention, la purification de la charge oléfinique, en particulier l'élimination simultanée de l'isobutanal et de l'éthanol, est réalisée dans un réacteur comprenant plusieurs lits fixes placés en parallèle et permutables. Ainsi, il est possible de soustraire un des lits de garde à des fins de régénération lorsque le ou les adsorbants le constituant sont saturés en impuretés, ceci sans arrêt du débit de la charge et donc sans arrêt de production.

Plusieurs options sont envisageables pour la phase de régénération du lit fixe ou lit de garde saturé.

Selon un premier mode de réalisation, le lit de garde saturé peut être extrait du réacteur. Dans ce cas, on peut avantageusement éliminer le liquide, par exemple par stripage, puis le solide et nettoyer cette partie du réacteur. Le rechargement peut avantageusement être fait avec une nouvelle charge d'adsorbant, ou bien avec l'ancienne charge d'adsorbant régénérée, par exemple, par passage d'un solvant entre 30 et 200°C ou par brûlage, avant de replacer cette partie dans le réacteur.

Selon un deuxième mode de réalisation, la régénération du lit d'adsorbant saturé peut avantageusement se faire par rinçage en ligne avec un solvant à co- ou contre-courant pour désorber les impuretés adsorbées à l'étape d'adsorption. Le solvant est avantageusement un hydrocarbure léger choisi parmi le pentane, l'heptane ou l'hexane. Selon un mode de réalisation de l'invention, le solvant peut être chauffé à une température comprise entre 30 et 350°C. La pression est comprise entre 0,1 et 10 MPa, de préférence, entre 0,3 et 5 MPa. Après une séparation en sortie de réacteur des impuretés adsorbées lors de la phase d'adsorption et extraites lors de cette régénération de l'adsorbant, et du solvant de régénération, le solvant est avantageusement recyclé dans le réacteur pour poursuivre la régénération.

Selon un troisième mode de réalisation, la phase de régénération du lit de garde saturé en impuretés est avantageusement réalisée par passage d'un gaz entre 180 et 500°C. Le gaz utilisé peut être un gaz de combustion, de l'air, de l'azote. De préférence le gaz de régénération est l'azote. Selon une variante de l'invention, le gaz de régénération peut contenir de l'eau, entre quelques ppm volumiques et 40% volumique d'eau, de préférence entre 0,1 et 20% volumique et de manière encore plus préférée entre 0,1 et 10% volumique.

De manière préférée, la régénération de l'adsorbant se fait in situ. De manière encore plus préférée, la régénération de l'adsorption se fait in situ avec un gaz chaud.

Dans le cas où la purification selon l'invention est mise en œuvre en lit fixe permutable d'au moins un adsorbant comprenant un matériau à base d'oxyde réfractaire poreux, la permutation est avantageusement réalisée lorsque la teneur en impuretés hydrocarbonées oxygénées, dans le mélange de butène après passage sur l'adsorbant comprenant au moins un matériau à base d'oxyde réfractaire est supérieure à 10 ppm massique, de manière préférée supérieure à 5 ppm massique et de manière encore plus préférée supérieure à 1 ppm massique.

De préférence, ledit procédé de purification d'oléfines selon l'invention est utilisé en amont de tout procédé ou toute étape de transformation d'oléfines. Dans un mode de réalisation préféré, ledit procédé de purification peut être placé en amont d'un procédé de métathèse desdites oléfines. Dans le cas où ledit procédé de purification est placé en amont d'un procédé de métathèse, le mélange d'oléfines purifié constitue la charge du procédé de métathèse.

Les exemples qui suivent sont présentés à titre illustratif et non limitatif.

### Description des Figures

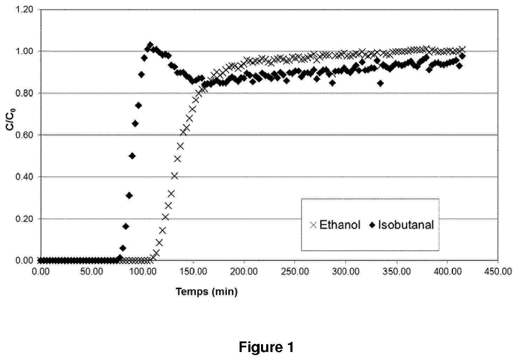

La Figure 1 présente les courbes de perçage de l'éthanol et de l'isobutanal obtenues lors de l'essai de co-adsorption sur l'adsorbant préparé dans l'exemple A et dans les conditions du test 2 du Tableau 1.

### Exemples:

Dans les exemples B et D ci-dessous, la charge oléfinique a été préalablement strippée à l'azote puis passée sur un tamis 3A. A l'issue de ce pré-traitement, les teneurs en eau et acétone de la charge sont vérifiées respectivement par la méthode de Karl Fischer et par chromatographie en phase gazeuse selon la méthode UOP 960. La charge oléfinique qui est testée dans les exemples B et D ne contient plus ni eau et ni acétone.

Dans l'exemple C ci-dessous, la charge oléfinique a été préalablement passée sur un tamis 3A. Une analyse par la méthode de Karl Fischer montre qu'à l'issue de ce pré-traitement, la charge oléfinique testée dans l'exemple C ne contient plus d'eau.

### Exemple A. Préparation d'un adsorbant selon l'invention

L'adsorbant est préparé par imprégnation à sec d'un matériau de type alumine flash, mis en forme par granulation, par une solution de NaOH

L'alumine choisie possède un volume poreux de 0,5 ml/g, un volume macroporeux de 0,1 ml/g, une surface spécifique de 341 m²/g et une teneur en sodium avant imprégnation de 1948 ppm massique.

La surface spécifique est déterminée par la méthode B.E.T selon la norme ASTM D3663. Le volume poreux total et le volume macroporeux sont déterminés par application de la norme ASTM D4284 à la mesure d'intrusion au mercure avec un angle de mouillage de 140°, au moyen d'un appareil modèle Autopore III™ de la marque Microméritics™ La teneur en sodium est mesurée par spectroscopie d'absorption atomique.

L'imprégnation est réalisée de la manière suivante :
a) préparation de 50 mL de solution de soude (NaOH) par dissolution de 8,84 g de NaOH dans 50 g d'eau ;
b) écoulement, au goutte à goutte à l'aide d'une burette, de 5 mL de la solution de soude préparée selon à l'étape a), sur 10 g d'alumine flash placée préalablement dans un drageoir tournant (phase d'imprégnation) ;
c) maturation du matériau imprégné dans une enceinte close saturée en eau à 20°C, pendant 3 h ;
d) séchage du solide sous air sec à 90°C pendant 3h dans une étuve ;
e) calcination du solide sous air sec à 350°C pendant 1h.

Après imprégnation, la teneur en sodium mesurée par spectroscopie d'absorption atomique est de 4,1% poids par rapport au poids total de l'adsorbant.

L'adsorbant A préparé est de couleur blanche.

### Exemple B. Co-adsorption d'isobutanal et d'éthanol et production de butènes purifiés

L'adsorbant A préparé selon l'exemple A est testé dans un réacteur en lit fixe, à 30°C sous 0,8 MPa. La charge est composée de 80% de n-butène, de 20% d'isobutène, et comprend de l'isobutanal et de l'éthanol en proportions variables (cf. Tableau 1, Tests de 1 à 3).

Parallèlement, un adsorbant de type zéolithe NaY de chez Zeolyst, de couleur blanche, mis en forme par pastillage et concassage, est testé dans les mêmes conditions. La charge est composée de 80% de n-butène, de 20% d'isobutène, et comprend de l'isobutanal et de l'éthanol à hauteur respectivement de 3023 et 963 ppm massique.

Le lit fixe d'adsorbant est préalablement activé à 290°C sous N₂ pendant 12h puis est rempli de butane à 30°C sous 0,8 MPa. La charge est ensuite injectée dans le réacteur, à un débit de 0,5 ml/min en continu, soit une VVH de 0,5 h⁻¹.

Le suivi des concentrations de l'isobutanal et de l'éthanol en sortie de réacteur est réalisé grâce à une analyse chromatographique en phase gaz sur un microGC 4900 de chez Varian.

Le test est arrêté quand la concentration en impuretés isobutanal et éthanol de l'effluent de sortie est la même que celle de la charge en entrée.

En fin de test, l'adsorbant A et la zéolithe NaY sont de couleur blanche.

Les capacités de captation des adsorbants à saturation en isobutanal et en éthanol sont calculées par bilan matière entre ce qui entre et ce qui sort de la colonne. Ces capacités de captation, exprimées en g de l'impureté considérée (isobutanal ou éthanol) pour 100 g d'adsorbant, sont reportées dans le Tableau 1 ainsi que les concentrations de la charge en isobutanal et éthanol, exprimées en ppm massique et leur rapport molaire.

**Tableau 1 : Quantités en isobutanal et en éthanol de la charge et quantités adsorbées par l'adsorbant A (alumine dopée au sodium) et par la zéolithe NaY**

| Adsorbant | Test | [Isobutanal] (ppm massique) | [Ethanol] (ppm massique) | Rapport molaire isobutanal/éthanol | q _{ads_isobutanal} (g/100g) | q_{ads_éthanol} (g/100g) |
|---|---|---|---|---|---|---|
| **A** (selon l'invention) | 1 | 1796 | 330 | 3,5 | 6,4 | 2,2 |
| | 2 | 644 | 410 | 1 | 4,3 | 3,4 |
| | 3 | 9 | 30 | 0,2 | 2,9 | 5,2 |
| **NaY** (test comparatif) | 4 | 13023 | 963 | 2 | 1,4 | 18,4 |

L'adsorbant A possède des capacités de captation importantes en isobutanal et en éthanol et du même ordre de grandeur quelles que soient les concentrations et les proportions d'isobutanal et d'éthanol dans la charge. Même dans le cas d'une charge avec un excès d'éthanol par rapport à l'isobutanal (rapport molaire isobutanal/éthanol à 0,2), la capacité de captation de l'adsorbant A en isobutanal reste élevée (2,9 g pour 100 g d'adsorbant).

A l'inverse, la zéolithe NaY montre une capacité de captation de l'isobutanal faible (1,4 g pour 100 g d'adsorbant) lorsque la charge contient de l'éthanol, même avec un excès en isobutanal (2 fois plus d'isobutanal que d'éthanol). Cet adsorbant n'est donc pas adapté pour réaliser l'élimination conjointe d'isobutanal et d'éthanol.

Les courbes de perçage de l'éthanol et de l'isobutanal sur l'adsorbant préparé dans l'exemple A, obtenues lors de l'essai de co-adsorption de l'éthanol et de l'isobutanal à iso-concentration molaire dans la charge (test 2 du Tableau 1) sont représentées sur la Figure 1.

D'après la Figure 1, il apparait que le procédé selon l'invention, qui est réalisé sur un lit d'adsorbant A (alumine imprégnée de sodium) et à 30°C, permet d'obtenir des teneurs en isobutanal et éthanol dans l'effluent de sortie inférieures à 1 ppm massique. En effet, les concentrations en isobutanal et éthanol dans l'effluent en sortie de réacteur sont inférieures à la limite de détection de 1 ppm massique pendant 75 et 105 minutes respectivement pour l'isobutanal et l'éthanol.

### Exemple C. Co-adsorption d'isobutanal, d'éthanol et d'acétone et production de butènes purifiés - exemple comparatif

Un test similaire à celui de l'exemple B est mené avec une charge composée de 80% de n-butène, de 20% d'isobutène, et comprenant un mélange d'isobutanal (642 ppm massique), d'éthanol (413 ppm massique) et d'acétone (519 ppm massique).

Après le test, l'adsorbant A est déchargé. Il est de couleur orange.

La comparaison de l'aspect de l'adsorbant A après les tests, entre l'exemple B (charge comprenant de l'isobutanal et de l'éthanol) et l'exemple C (charge comprenant de l'isobutanal, de l'éthanol et de l'acétone), démontre que la présence d'acétone dans la charge a entraîné la dégradation de celle-ci au cours du test. Une charge contenant de l'acétone doit donc être pré-traitée pour éliminer au moins l'acétone, préalablement à l'étape d'élimination conjointe sur un adsorbant selon l'invention de l'isobutanal et de l'éthanol.

### Exemple D. Régénérabilité de l'adsorbant et cyclage

L'adsorbant A préparé selon l'exemple A est testé sur lit fixe dans une colonne, à 30°C sous 0,8 MPa. La charge est composée de 80% de n-butène, de 20% d'isobutène et comprend 644 ppm massique d'isobutanal et 410 ppm massique d'éthanol. Les conditions opératoires suivies sont celles décrites dans l'exemple B.

Les concentrations en isobutanal et en éthanol dans l'effluent de sortie est suivie par analyse chromatographique en phase gaz sur un microGC 4900 de chez Varian.

Quand la composition en sortie devient identique à celle en entrée, l'adsorbant est considéré comme saturé. Le débit de la charge est stoppé et l'adsorbant est régénéré sous flux d'azote à pression atmosphérique à 290°C à 20 NL/h. Quand les concentrations en isobutanal et en éthanol en sortie de colonne deviennent nulles, la régénération est considérée comme terminée. Le flux d'azote est remplacé par un débit de charge identique à celle du 1^{er} cycle de manière à procéder à un deuxième cycle d'adsorption. Les concentrations de l'isobutanal et de l'éthanol en sortie de réacteur sont suivies comme précédemment par analyse chromatographique en phase gaz sur un microGC 4900. Quand la composition en sortie devient identique à celle en entrée, la colonne est de nouveau soumise à un flux d'azote à pression atmosphérique à 290°C. 4 cycles d'adsorption/régénération sont enchainés. Les capacités de captation en isobutanal et en éthanol sont reportées dans le Tableau 2.

**Tableau 2 : Quantités adsorbées en isobutanal et éthanol par l'adsorbant A après chaque cycle d'adsorption/désorption**

| Cycle | q_{ads_isobutanal} (g/100g) | q_{ads_éthanol} (g/100g) |
|---|---|---|
| 1 | 4,3 | 3,4 |
| 2 | 3,5 | 3,0 |
| 3 | 2,5 | 2,4 |
| 4 | 1,8 | 2,2 |
| 5 | 1,6 | 2,1 |

Les capacités de captation en isobutanal et éthanol restent satisfaisantes après 5 cycles. L'adsorbant A est donc toujours en capacité d'éliminer conjointement l'isobutanal et l'éthanol après 5 cycles.

## Revendications

1. Procédé de purification d'une charge oléfinique comprenant des oléfines à 4 atomes de carbone et des impuretés incluant l'isobutanal, l'éthanol et l'acétone, ledit procédé comprenant :
a) une étape de pré-traitement comprenant au moins une étape d'élimination de l'acétone ; et
b) une étape d'élimination simultanée de l'isobutanal et de l'éthanol, par passage de la charge pré-traitée issue de l'étape a) sur au moins un lit fixe d'au moins un adsorbant comprenant au moins un matériau à base d'oxyde réfractaire poreux,
ladite étape b) opérant à une température comprise entre 0 et 200°C, à une pression de 0,1 à 10 MPa et avec une vitesse volumique horaire (VVH) de la charge pré-traitée issue de l'étape a) sur le lit fixe comprise entre 0,1 et 10 h⁻¹.

2. Procédé selon la revendication 1, dans lequel ladite charge oléfinique est issue de la déshydratation de l'isobutanol ou d'un mélange d'isomères du butanol comprenant l'isobutanol.

3. Procédé selon l'une des revendications précédentes, dans lequel l'étape de pré-traitement comprend en outre une phase d'élimination de l'eau présente dans ladite charge oléfinique, les phases d'élimination de l'acétone et d'élimination de l'eau étant réalisées simultanément ou successivement.

4. Procédé selon l'une des revendications précédentes, dans lequel le matériau à base d'oxyde réfractaire poreux est choisi dans le groupe constitué par : l'alumine, la silice, l'oxyde de titane, l'oxyde de zirconium, l'oxyde de magnésium et leurs mélanges.

5. Procédé selon l'une des revendications précédentes, dans lequel le matériau à base d'oxyde réfractaire poreux est à base d'alumine.

6. Procédé selon l'une quelconque des revendications précédentes, dans lequel le matériau à base d'oxyde réfractaire poreux présente un volume macroporeux, c'est-à-dire un volume cumulé des pores de diamètre supérieurs à 50 nm, mesuré par intrusion au mercure, supérieur ou égal à 0,01 ml/g, un volume poreux total, mesuré par intrusion au mercure selon la norme ASTM D4284 avec un angle de mouillage de 140°, supérieur ou égal à 0,05 ml/g et une surface spécifique, exprimée en S_{BET} et mesurée par la méthode B.E.T selon la norme ASTM D3663, comprise entre 30 m²/g et 400 m²/g.

7. Procédé selon l'une des revendications précédentes, dans lequel le matériau à base d'oxyde réfractaire poreux est imprégné d'un ou plusieurs cations alcalins ou alcalino-terreux.

8. Procédé selon la revendication précédente, dans lequel le cation est un cation d'un élément choisi dans le groupe constitué par : le sodium, le potassium, le magnésium et le calcium.

9. Procédé selon l'une des revendications 7 et 8, dans lequel le matériau à base d'oxyde réfractaire poreux est imprégné d'un ou plusieurs cations alcalins ou alcalino-terreux à hauteur de 0,2% à 40% poids, par rapport au poids total de l'adsorbant après imprégnation.

10. Procédé selon l'une quelconque des revendications précédentes, dans lequel l'étape b) opère à une température comprise entre 20 et 60°C.

11. Procédé selon l'une quelconque des revendications précédentes, dans lequel l'étape b) opère à une pression comprise entre 0,3 et 5 MPa.

12. Procédé selon l'une quelconque des revendications précédentes, dans lequel l'étape b) est conduite telle que la vitesse volumique horaire (VVH) de ladite charge sur ledit lit fixe est comprise 0,2 et 5 h⁻¹.

13. Procédé selon l'une quelconque des revendications précédentes, dans lequel l'étape b) est réalisée dans un réacteur comprenant plusieurs lits fixes placés en parallèle et permutables.

14. Procédé de transformation d'oléfines comprenant une étape correspondant au procédé de purification de la charge oléfinique selon l'une des revendications précédentes en amont du procédé de métathèse.

## Patentansprüche

1. Verfahren zur Reinigung einer Olefin-Beschickung, die Olefine mit 4 Kohlenstoffatomen und Verunreinigungen, einschließlich Isobutanal, Ethanol und Aceton, umfasst, wobei das Verfahren Folgendes umfasst:
a) einen Vorbehandlungsschritt, der mindestens einen Acetonbeseitigungsschritt umfasst; und
b) einen Schritt der gleichzeitigen Beseitigung von Isobutanal und Ethanol mittels Leiten der vorbehandelten Beschickung aus Schritt a) über mindestens ein Festbett mindestens eines Adsorptionsmittels, das mindestens ein poröses Material auf der Basis von feuerfestem Oxid umfasst,
wobei Schritt b) bei einer Temperatur zwischen 0 und 200°C, bei einem Druck von 0,1 bis 10 MPa und mit einer Raumstundengeschwindigkeit (VVH) der vorbehandelten Beschickung aus Schritt a) über dem Festbett zwischen 0,1 und 10 h⁻¹ erfolgt.

2. Verfahren nach Anspruch 1, wobei die Olefin-Beschickung aus der Dehydratisierung von Isobutanol oder eines Gemischs von Isomeren von Butanol, das Isobutanol umfasst, herrührt.

3. Verfahren nach einem der vorhergehenden Ansprüche, wobei der Vorbehandlungsschritt außerdem eine Phase der Beseitigung des in der Olefin-Beschickung vorhandenen Wassers umfasst, wobei die Phasen der Beseitigung von Aceton und der Beseitigung von Wasser gleichzeitig oder nacheinander durchgeführt werden.

4. Verfahren nach einem der vorhergehenden Ansprüche, wobei das poröse Material auf der Basis von feuerfestem Oxid aus der Gruppe ausgewählt ist, bestehend aus: Aluminiumoxid, Siliziumdioxid, Titanoxid, Zirkonoxid, Magnesiumoxid und deren Gemischen.

5. Verfahren nach einem der vorhergehenden Ansprüche, wobei das poröse Material auf der Basis von feuerfestem Oxid auf Aluminiumoxid basiert.

6. Verfahren nach einem der vorhergehenden Ansprüche, wobei das poröse Material auf der Basis von feuerfestem Oxid ein Makroporenvolumen, d. h. ein mittels Quecksilberintrusion gemessenes kumulatives Volumen von Poren mit einem Durchmesser von mehr als 50 nm, größer als oder gleich 0,01 ml/g, ein mittels Quecksilberintrusion gemäß der Norm ASTM D4284 mit einem Kontaktwinkel von 140° gemessenes Gesamt-Porenvolumen größer als oder gleich 0,05 ml/g und eine als S_{BET} ausgedrückte und durch das B.E.T.-Verfahren gemäß der Norm ASTM D3663 gemessene spezifische Oberfläche zwischen 30 m²/g und 400 m²/g aufweist.

7. Verfahren nach einem der vorhergehenden Ansprüche, wobei das poröse Material auf der Basis von feuerfestem Oxid mit einem oder mehreren Alkali- oder Erdalkalikationen imprägniert wird.

8. Verfahren nach dem vorhergehenden Anspruch, wobei es sich bei dem Kation um ein Kation eines Elements handelt, ausgewählt aus der Gruppe, bestehend aus: Natrium, Kalium, Magnesium und Calcium.

9. Verfahren nach einem der Ansprüche 7 und 8, wobei das poröse Material auf der Basis von feuerfestem Oxid mit einem oder mehreren Alkali- oder Erdalkalikationen zu 0,2 bis 40 Gew.-%, bezogen auf das Gesamtgewicht des Adsorptionsmittels nach der Imprägnierung, imprägniert wird.

10. Verfahren nach einem der vorhergehenden Ansprüche, wobei Schritt b) bei einer Temperatur zwischen 20 und 60°C erfolgt.

11. Verfahren nach einem der vorhergehenden Ansprüche, wobei Schritt b) bei einem Druck zwischen 0,3 und 5 MPa erfolgt.

12. Verfahren nach einem der vorhergehenden Ansprüche, wobei Schritt b) derart durchgeführt wird, dass das Raumstundenvolumen (VVH) der Beschickung über dem Festbett zwischen 0,2 und 5 h⁻¹ beträgt.

13. Verfahren nach einem der vorhergehenden Ansprüche, wobei Schritt b) in einem Reaktor durchgeführt wird, der mehrere parallel angeordnete und umschaltbare Festbetten umfasst.

14. Verfahren zur Umwandlung von Olefinen, umfassend einen Schritt, der dem Verfahren zur Reinigung der Olefin-Beschickung nach einem der vorhergehenden Ansprüche entspricht, stromaufwärts des Metatheseverfahrens.

## Claims

1. Method for purifying an olefinic feedstock that comprises olefins with 4 carbon atoms and impurities including isobutanal, ethanol, and acetone, where said method comprises:
a) A pretreatment step that comprises at least one acetone elimination step; and
b) A step for simultaneously eliminating isobutanal and ethanol by running the pretreated feedstock obtained from Step a) over at least one fixed bed with at least one adsorbent that comprises at least one porous refractory oxide-based material,
where said Step b) operates at a temperature of between 0 and 200°C, at a pressure of 0.1 to 10 MPa, and with an hourly volumetric flow rate (VVH) of the pretreated feedstock obtained from Step a) over the fixed bed of between 0.1 and 10 h⁻¹.

2. Method in accordance with Claim 1, in which said olefinic feedstock is produced from the dehydration of isobutanol or a mixture of butanol isomers that comprises isobutanol.

3. Method in accordance with one of the preceding claims, in which the pretreatment step also comprises a phase for eliminating the water that is present in said olefinic feedstock, where the acetone-elimination and water-elimination phases are carried out simultaneously or successively.

4. Method in accordance with one of the preceding claims, in which the porous refractory oxide-based material is selected from the group made up of: alumina, silica, titanium oxide, zirconium oxide, magnesium oxide, and mixtures thereof.

5. Method in accordance with one of the preceding claims, in which the porous refractory oxide-based material is based on alumina.

6. Method in accordance with any one of the preceding claims, in which the porous refractory oxide-based material has a macropore volume, i.e., a cumulative volume of the pores that are greater than 50 nm in diameter, as measured by mercury intrusion, of greater than or equal to 0.01 ml/g, a total pore volume, as measured by mercury intrusion according to the Standard ASTM D4284 with a wetting angle of 140°, of greater than or equal to 0.05 ml/g, and a specific surface area, expressed in terms of S_{BET} and measured by the B.E.T. method in accordance with the Standard ASTM D3663, of between 30 m²/g and 400 m²/g.

7. Method in accordance with one of the preceding claims, in which the porous refractory oxide-based material is impregnated with one or more alkaline or alkaline-earth cations.

8. Method in accordance with the preceding claim, in which the cation is a cation of an element that is selected from the group made up of: sodium, potassium, magnesium, and calcium.

9. Method in accordance with one of Claims 7 and 8, in which the porous refractory oxide-based material is impregnated with one or more alkaline or alkaline-earth cations at a level of 0.2 to 40% by weight, relative to the total weight of the adsorbent after impregnation.

10. Method in accordance with any one of the preceding claims, in which Step b) operates at a temperature of between 20 and 60°C.

11. Method in accordance with any one of the preceding claims, in which Step b) operates at a pressure of between 0.3 and 5 MPa.

12. Method in accordance with any one of the preceding claims, in which Step b) is carried out such that the hourly volumetric flow rate (VVH) of said feedstock on said fixed bed is between 0.2 and 5 h⁻¹.

13. Method in accordance with any one of the preceding claims, in which Step b) is carried out in a reactor that comprises multiple fixed beds that are arranged in parallel and that can be shuffled.

14. Method for transforming olefins that comprises a step corresponding to the method for purification of the olefinic feedstock in accordance with one of the preceding claims upstream from the metathesis method.
